(19) Europäisches Patentamt
European Patent Office
Office européen des brevets

(11) **EP 4 124 357 A1**

(12) **EUROPEAN PATENT APPLICATION**

(43) Date of publication:
**01.02.2023 Bulletin 2023/05**

(21) Application number: **22382017.6**

(22) Date of filing: **14.01.2022**

(51) International Patent Classification (IPC):
**A61N 1/05** (2006.01)     **A61B 5/293** (2021.01)
**A61B 5/00** (2006.01)

(52) Cooperative Patent Classification (CPC):
**A61N 1/0551; A61N 1/0531**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.07.2021 EP 21188748**

(71) Applicants:
• **Fundació Institut Català de Nanociència i
Nanotecnologia (ICN2)
08193 Cerdanyola del Vallès (ES)**
• **Institució Catalana De Recerca I
Estudis Avançats (ICREA)
08010 Barcelona (ES)**

• **Consejo Superior de Investigaciones Científicas
(CSIC)
28006 Madrid (ES)**
• **Consorcio Centro de Investigación
Biomédica en Red M.P (CIBER)
28029 Madrid (ES)**

(72) Inventors:
• **GARRIDO ARIZA, José Antonio
08960 Sant Just Desvern (ES)**
• **GUIMERÀ BRUNET, Antón
28006 Madrid (ES)**
• **ILLA VILA, Xavier
28029 Madrid (ES)**
• **GARCÍA CORTADELLA, Ramón
08193 Cerdanyola del Vallès (ES)**

(74) Representative: **Ponti & Partners, S.L.P
C. de Consell de Cent 322
08007 Barcelona (ES)**

(54) **A FLEXIBLE PROBE**

(57)     The present invention relates to a flexible probe, comprising:
- an electrically insulating flexible support comprising at least one shank portion (S) that comprises at least a first electrically insulating layer (PI) with a top face and a bottom face;
- a plurality of active elements arranged on the top face of the first electrically insulating layer (PI);
- a plurality of tracks (T) electrically connected to terminals of said plurality of active elements, wherein at least one electrically conductive track is arranged on the bottom face of the first electrically insulating layer (PI), and is electrically connected to a terminal of at least one of the plurality of active elements through a via hole defined in at least the first electrically insulating layer (PI); and
- at least part of the plurality of electrically conductive tracks (T) are electrically interconnected to implement a multiplexing technique.

Figure 1

**Description**

"The work leading to this patent application has received funding from the European Union's Horizon 2020 Research and Innovation Programme under Grant Agreements No. 649953 (Graphene Flagship) and No. 732032 (Brain-Com)."

FIELD OF THE INVENTION

The present invention relates to a flexible probe, where active elements are interconnected in order to implement a multiplexing technique, allowing to implement arrays with high aspect ratios, reducing the probe and connector footprints while integrating an increased number of active elements.

BACKGROUND OF THE INVENTION

Increasing the number of sensors and actuators integrated in any flexible probes, such as thin-film probes, is an important objective in biomedical technology, and in particular in neurotechnology for the monitoring and control of neural activity with high spatial resolution. Currently, the main bottleneck in the scalability of thin-film probes is the number of wires required to address each of the sensors and actuators. In the last decade, multiple strategies have been proposed to increase the number of sensors and actuators on thin-film devices by multiplexing their output signal or driving voltage supply(1-5).

These techniques allow to integrate an increased number of sensors or actuators in a given area and reduce the footprint and complexity of the connectors by increasing the number of elements addressed per contact. However, addressable active matrixes present an optimal reduction of wires for an equal number of columns (m) and rows (n). Therefore, they are sub-optimal for arrays with high aspect ratios, i.e. different number of columns and rows (m<n or n<m). Such devices present multiple limitations for their implantation in the body; having an aspect ratio close to one implies significant mechanical stress when conforming with surfaces curved in two dimensions, e.g. the brain surface. Furthermore, multiplexed probes with an aspect ratio close to one are not adequate to penetrate in the biological tissue, because penetrating probes must present a minimal cross-section to minimize tissue damage and the foreign body response (FBR) (6-9). Similarly, deep-brain stimulation (DBS) probes consist of an array of sensors/actuators on the surface of a cylinder with a diameter in the millimetre scale and a length of several centimetres. Rolling and attaching thin-film devices on DBS probes is possible, however multiplexed active arrays with an aspect ratio close to one are not effective in providing high sensor/stimulator counts.

It is therefore necessary to provide an alternative to the state of the art which covers the gaps found therein, by providing a flexible probe allowing to implement multiplexed active matrixes to increase the number of sensors and/or actuators in an active area of the probe with a high aspect ratio. For this objective, alternative strategies for the routing of the interconnection tracks are required, which allow the implementation of epicortical neural probes with one or multiple strips as well as depth probes (such as DBS or intracortical probes) increasing their spatial resolution while improving their conformability with curved surfaces and/or minimizing their cross-section and footprint, therefore minimizing their invasiveness.

SUMMARY OF THE INVENTION

To that end, the present invention relates to a flexible probe, comprising:

- an electrically insulating flexible support comprising at least one shank portion;
- a plurality of active elements arranged on said at least one shank portion; and
- a plurality of electrically conductive tracks electrically connected to terminals of said plurality of active elements.

In contrast to the flexible probes of the prior art, in the one of the present invention, in a characterizing manner:

- the at least one shank portion comprises at least a first electrically insulating layer with a top face and a bottom face, wherein the active elements of said plurality of elements are arranged on said top face of said first electrically insulating layer; and
- at least one electrically conductive track of said plurality of electrically conductive tracks is arranged on said bottom face of the first electrically insulating layer, running at a first depth level, and is electrically connected to a terminal of at least one of said plurality of active elements through a via hole defined in at least the first electrically insulating layer; and
- at least part of the plurality of electrically conductive tracks are electrically interconnected to implement a multiplexing technique.

The term "active elements" stands for electronic devices that require a voltage supply, such as transistors that can operate as active sensing elements as well as switching elements.

For an embodiment, the at least part of the plurality of electrically conductive tracks (T) are electrically interconnected so that with m + n electrically conductive tracks (T) the active elements are addressed in an $(m \times j) \times (n / j)$ array, where n is a multiple of j, where m, n and j are positive integers.

In other words, a $m \times n$ addressable array (where m an n can have the same value, e.g. m=n=32) is rearranged into an array with the active elements distributed in an array with a high aspect ratio (e.g. 32x8 $\times$ 32/8 = 256 $\times$ 4), where all the active elements can be addressed selectively by selecting one column and one one row from the m columns and n rows respectively. In this way, a high number of active elements (e.g. 1024) in an array with a high aspect ratio (e.g. 256 $\times$ 4 = 1024) can be addressed using a reduced number of conductive tracks (e.g. 32 + 32 = 64) compared to state of the art multiplexed probes (which would require a larger number of conductive tracks, following the example 256 + 4 = 260).

According to an embodiment, the at least one shank portion comprises a stack of electrically insulating layers, including at least said first electrically insulating layer on top of at least a second electrically insulating layer.

For an embodiment, the flexible probe of the present invention is a thin-film probe., while for other embodiments, the flexible is manufactured according to a process which is not based on thin-film technology.

For an embodiment, the electrically insulating flexible support comprises at least one further shank portion, and wherein the flexible probe further comprises:

- a further plurality of active elements arranged on the further shank portion; and
- a further plurality of electrically conductive tracks electrically connected to terminals of the further plurality of active elements;

wherein:

- the at least one further shank portion comprises at least a first further electrically insulating layer with a top face and a bottom face, wherein the active elements of said further plurality of active elements are arranged on said top face of said first further electrically insulating layer;
- at least one electrically conductive track of said further plurality of electrically conductive tracks is arranged on said bottom face of the first further electrically insulating layer), running at a first depth level, and is electrically connected to a terminal of at least one of said further plurality of active elements through a via hole defined in at least the first further electrically insulating layer; and
- at least part of the further plurality of electrically conductive tracks are electrically interconnected to implement a multiplexing technique.

For an implementation of that embodiment, the at least one further shank portion comprises a stack of electrically insulating layers, including at least said first further electrically insulating layer on top of at least a second further electrically insulating layer.

Preferably, the stacks of all the shanks portions, including the at least one shank portion and the at least one further shank portion, of flexible probe of the present invention have the same number of layers, both electrically insulating layers and also electrically conductive layers (where the electrically conductive tracks are defined).

According to an embodiment, at least part of the plurality of electrically conductive tracks are interconnected with at least part of the further plurality of electrically conductive tracks, to implement a multiplexing technique.

For an implementation of that embodiment, the electrically insulating flexible support comprises a base portion from which the at least one shank portion and the at least one further shank portion project, wherein the interconnection of at least part of the plurality of electrically conductive tracks with at least part of the further plurality of electrically conductive tracks is made on the base portion.

Here, for the above mentioned embodiment, the probe of the present invention uses multiplexed active matrixes implemented in multiple shanks, or shank portions, each containing multiple active elements. In order to lay out the proper connectivity among active elements, multiple insulated metal lines are stacked. For that embodiment, the multiplexing is performed at two different levels; first, within each particular shank and secondly, among shanks by interconnecting the shared metal lines at the base of the flexible probe, i.e. at the above mentioned base portion. This strategy allows to increase the number of sensors in flexible probes while maximizing their aspect ratio and minimizing the footprint of the connectors. This concept has been demonstrated by the present inventors using graphene solution gated field-effect transistors, which present a high sensitivity in a wide frequency band and have been demonstrated effective for neural sensing(*1, 10, 11*). According to an embodiment, the at least one shank portion and/or the at least one further shank portion has a distal free end part and a proximal end part. The proximal end part is wider than the distal free end part, wherein the proximal end part is joined to the base portion, generally integral thereto. Generally, the purpose of the wider proximal end part is to ease the implantation of the flexible probe while keeping a low electrically conductive track resistance. I.e., it reduces the electrical resistance of long tracks that rut to the base portion.

For an implementation of that embodiment, the proximal end part that is from 2 to 20 times wider and from 2 to 10 times longer than the distal free end part.

For an embodiment, at least two electrically conductive tracks of the plurality and/or further plurality of electrically conductive tracks run, at the same depth level, said first depth level, and are electrically connected to at least two respective terminals of at least one active element of the plurality and/or of the further plurality of active elements through at least two respective via holes defined in at least the first electrically insulating layer and/or the first further electrically insulating layer.

According to an embodiment:

- the at least one electrically conductive track of the plurality of electrically conductive tracks runs at the first depth level, in the stack of the shank portion, and wherein at least another electrically conductive track of the plurality of electrically conductive tracks is arranged on a bottom face of the second electrically insulating layer, running at a second depth level in the stack of the shank portion, and is electrically connected to a terminal of at least one active element of the plurality of active elements through at least two via holes respectively defined in at least the first and second electrically insulating layers; and/or

- the at least one electrically conductive track of the further plurality of electrically conductive tracks runs at the first depth level, in the stack of the further shank portion, and wherein at least another electrically conductive track of the further plurality of electrically conductive tracks is arranged on a bottom face of the second further electrically insulating layer, running at a second depth level in the stack of the further shank portion, and is electrically connected to a terminal of at least one active element of the further plurality of active elements through at least two via holes respectively defined in at least the first and second further electrically insulating layers.

For an implementation of that embodiment, the flexible probe of the present invention further comprises at least one intermediate electrically conductive connection arranged, at the first depth level, between the first and second electrically insulating layers and/or between the first and second further electrically insulating layers, and which is electrically connected, through the at least two via holes, to the terminal of at least one active element and to the electrically conductive track running at the second depth level of the stack of the shank portion and/or of the further shank portion.

According to a variant of said implementation, the at least one intermediate electrically conductive connection is spatially shifted and electrically insulated from the at least one electrically conductive track also running at the first depth level.

For some embodiments, at least one of the electrically conductive tracks is arranged on the first electrically insulating layer (for the at least one shank portion) or on the first further electrically insulating layer (for the at least one further shank portion), i.e. at the same level (called below as zero depth level) as the active elements.

Although the present invention can constitute any kind of flexible probe, such as a radiation sensing probe configured to be implanted near an organ for sensing radiation applied thereto, for a preferred embodiment the flexible probe of the present invention constitutes an intracranial neural probe for either epicortical or depth electrical sensing and/or stimulation.

For an embodiment, the intracranial neural probe is an epicortical neural probe. For an implementation of that embodiment the shank portion and/or further shank portion represent one or multiple strip probes, each conforming with the curvature of the brain dominated by one dimension.

For another implementation of that embodiment the shank portion and/or further shank portion or the respective distal free end part thereof (in case, such a distal free part exists), has a width below 1.5 mm and a length ranging between 20 and 100 mm; and

- each electrically conductive track has an electrical resistance below 50 $\Omega$, a maximum thickness of 500 nm, a width ranging between 4.5 $\mu$m and 15 $\mu$m, and a separation of at least 3 $\mu$m with respect to another electrically conductive track.

For another embodiment, the intracranial neural probe is a depth neural probe, such as an intra-cortical or subcortical neural probe.

For an implementation of that embodiment, the shank portion and/or further shank portion, or the respective distal free end part thereof (in case, such a distal free part exists), present a minimal width to minimize tissue damage during insertion.

For a variant of said implementation:

- the shank portion and/or further shank portion, or the respective distal free end part thereof (in case, such a distal free part exists), has a width below 150 $\mu$m and a length ranging between 2 mm and 10 mm; and
- each electrically conductive track has an electrical resistance below 50 $\Omega$, a maximum thickness of 500 nm, a width ranging between 4.5 $\mu$m and 15 $\mu$m, and a separation of at least 3 $\mu$m with respect to another electrically conductive track.

For another embodiment, the intracranial neural probe is a flexible probe that can be rolled and/or attached on a three-dimensional structure such as a DBS probe or a catheter.

For an implementation of that embodiment, the flexible probe constitutes a depth neural probe, and the flexible support is dimensioned, shaped, and flexible enough so that it can be rolled around a three-dimensional structure, such as around the outer cylindrical wall of a tubular structure.

For an embodiment, multiple shank portions in different components of a three-dimensional structure are interconnected at the base of the flexible probe reducing the contacts required per shank.

According to a variant of any of those implementations of the intracranial neural probe, the shank portion and/or further shank portion has a width ranging from (4.5 $\mu$m +3 $\mu$m)·N and (15 $\mu$m +3 $\mu$m)·N, wherein N stands for the number of electrically conductive tracks per depth level in the stack, and, preferably, plus a space between 2 $\mu$m and 50 $\mu$m on either sides of the shank portion without electrically conductive tracks placed thereon, to ensure a good insulation between the electrically conductive tracks

According to an embodiment, the base portion also comprises a stack of at least first and second electrically insulating layers, and wherein the interconnection of at least part of the plurality of electrically conductive tracks with at least part of the further plurality of electrically conductive tracks is made by means of electrically interconnecting the same with interconnection electrically conductive tracks arranged at a depth level, in the base portion stack, that is different to the depth level at which the electrically conductive tracks run, in the shank portion and further shank portion, respectively.

According to an embodiment, the active elements are transistors, such as field effect transistors (for example g-SGFETs) or organic electrochemical transistors.

For an implementation of that embodiment, the drain and source terminals of the transistors are electrically connected to the electrically conductive tracks, so that the transistors are arranged in an addressable matrix with m columns and *n* rows, where the electrically conductive tracks for the m columns address the common drain or source terminal of all transistors in each column, and the *n* rows address either the common drain or source terminal or the common gate terminal of all transistors in each row, to implement the multiplexing technique.

For an embodiment, a maximum of one electrically conductive track implementing one of said m columns is arranged on the first electrically insulating layer (for the at least one shank portion) or on the first further electrically insulating layer (for the at least one further shank portion), i.e. at the same level (called below as zero depth level) as the sensing elements, and electrically connected to n transistor source terminals (one per row) in a direct manner (i.e. without any via hole), while the electrically conductive tracks of the rest of m columns are arranged at different depth levels inside the corresponding stacks of electrically insulating layers, each electrically connected to the source terminals of n other transistors (one per row) though corresponding via holes. a variant of that embodiment, some or all of the electrically conductive tracks implementing the n rows are arranged on the first electrically insulating layer (for the at least one shank portion) or on the first further electrically insulating layer (for the at least one further shank portion), i.e. at the same level as the sensing elements, while for another variant all the electrically conductive tracks implementing the n rows are arranged at different depth levels inside the corresponding stacks of electrically insulating layers.

For an implementation of that embodiment, the resistance of the metal tracks in the shanks must present values below 50$\Omega$ that lead to a signal-to-crosstalk ratio above 36 dB(1, 10), which is suitable for neural sensing with arrays where n+m < 12(*1, 10*). For larger arrays, the track resistance must be below 50 x12 /(n+m) $\Omega$. For an implementation of a base comprising the interconnection among k shanks, the electrical resistance of the interconnection conductive tracks is at least a k factor smaller than the resistance of the electrically conductive tracks, where k stands for the number of shank portions, including the shank portion and the further shank portion.

For some embodiments, in addition to the above mentioned active elements, the flexible probe of the present invention also includes passive elements (such as electrodes coupled to the gate of a transistor transducer).

BRIEF DESCRIPTION OF THE FIGURES

In the following some preferred embodiments of the invention will be described with reference to the enclosed figures. They are provided only for illustration purposes without however limiting the scope of the invention.

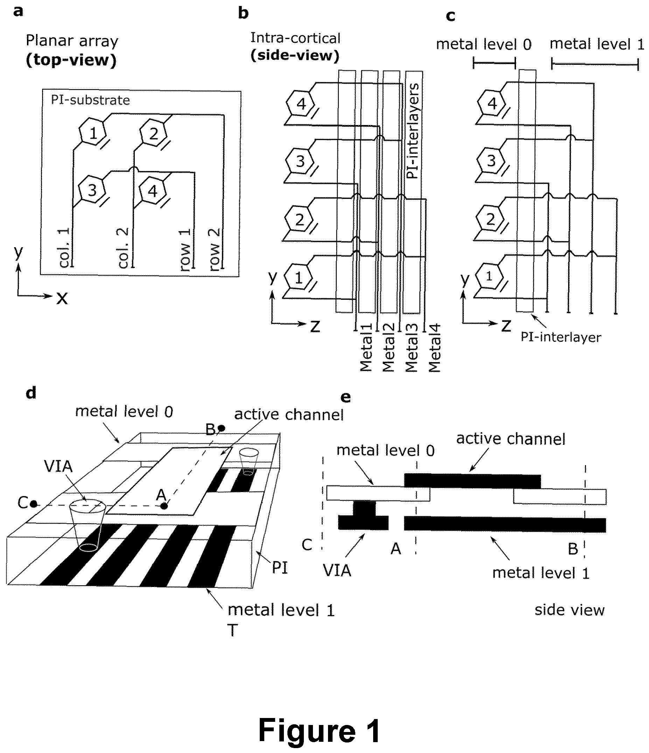

Figure 1 schematically shows comparison of state of the art probe with the present invention for different embodiments: a, state of the art 2x2 multiplexed active sensor array for surface recordings. b, (2x2) $\times$ (2/2) multiplexed

active sensor array, with one metal line per metal level, for intra-cortical recordings. c, (2x2) $\times$ (2/2) multiplexed active sensor array, with four metal lines in one metal level. d, Schematic of an active sensor on metal level 0 connected through VIAs to the metal lines in metal level 1. e, Side view of the device from point C to A and A to B labelled in part d.

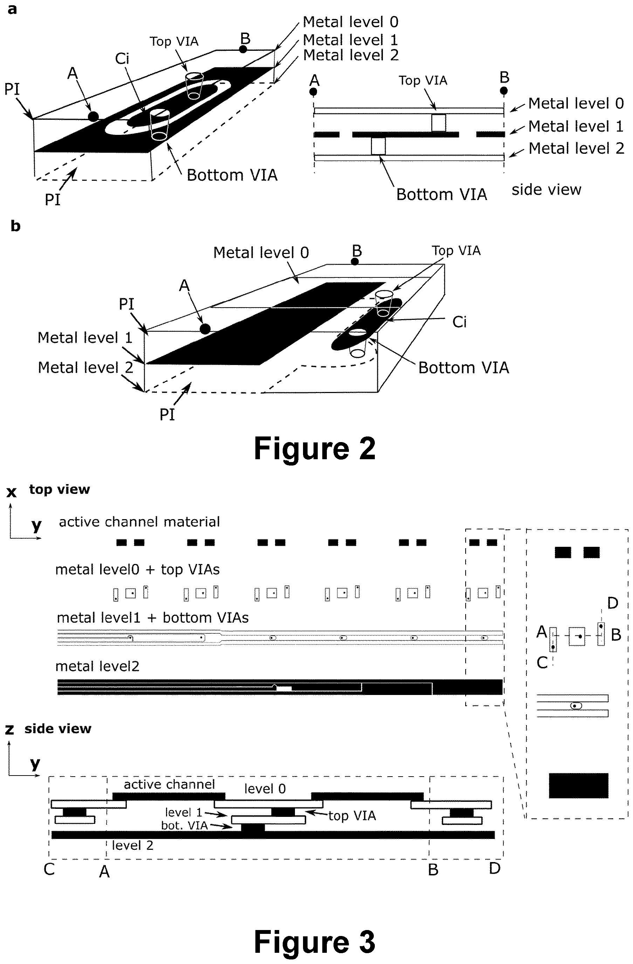

Figure 2 schematically shows part of the probe of the present invention for two embodiments which show different connection arrangements between the metal lines and connections placed at three different depth levels in the stack of the probe: a, top VIA and bottom VIA to connect the metal level 0 to metal level 2. An interconnection between VIAs is used in metal level 1, which takes space from metal lines at this level (left schematic). The side view between points A and B is shown (right schematic). b, Connection between the metal level 2 and metal level 0 with an intermediate connection at metal level 1, which is in next to and disconnected from the lines in metal level 1.

Figure 3 schematically shows different metal elements and active channels (of twelve corresponding transistors) arranged on different depth levels of a shank portion of the probe of the present invention, for an embodiment (the insulating electrically insulating layers separating the different metal levels have not been depicted, for clarity sake). Top view separately show the elements arranged on the different levels of the stack of the shank portion, and an enlarged view of a portion thereof. The metal level 0, level 1, level 2, top and bottom VIAs as well as active channel material layers are illustrated for a 2x6 array. Using the two central lines of the metal level 1 as rows allows to have their corresponding 2 sensors grouped closer to the base of the probe, leaving additional space in metal level 1 along the rest of the shank. This extra space can be used for VIAs from the rows in metal level 2. Using rows in metal level 2 the two sensors for each row can be again grouped, leaving more space in metal level 2 along the shank that can be used to align all the VIAs. The points A, B, C and D are defined on the zoomed schematic on the right. The side view between points C to A, A to B and B to D are shown on the bottom schematic.

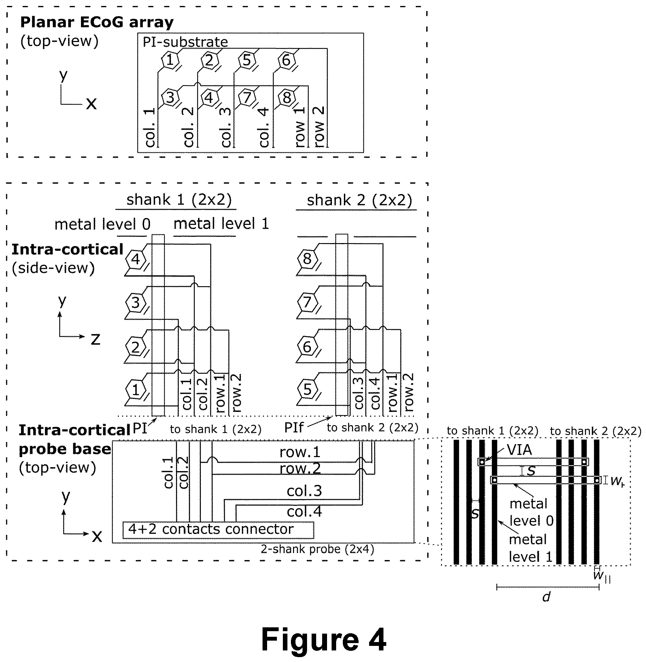

Figure 4 comparison of state of the art probe with the present invention for a particular embodiment: Top: state of the art 4x2 active sensor array for surface neural recordings. Bottom: an embodiment for implementing a 4x2 active sensor array distributed in two shanks (or shank portions). The 2 rows are common to both shanks. The interconnection of the rows from both shanks is carried out at the flexible base of the probe, before the connection to rigid electronics. The inset on the bottom-right shows the interconnections, which can be defined on metal level 0, and must be at a different level than the metal lines for columns and rows coming from the shanks.

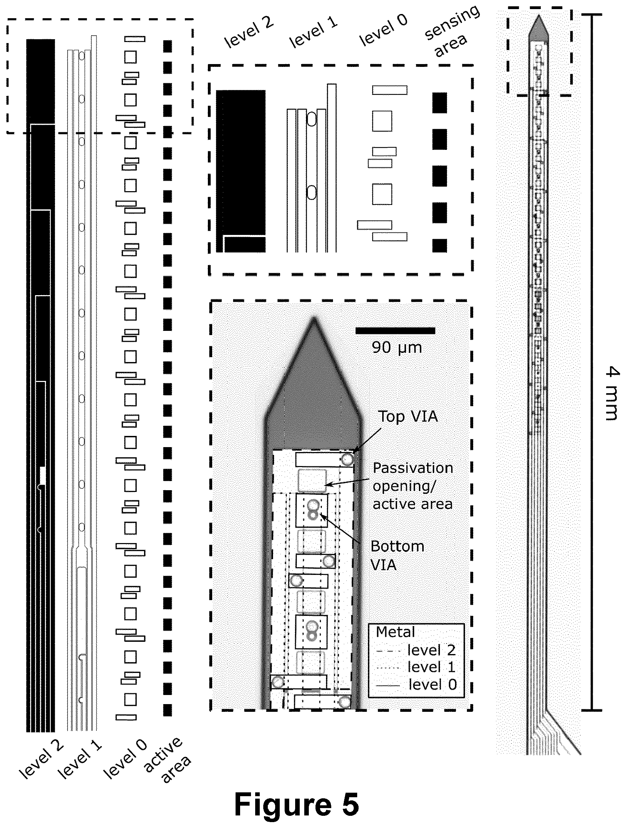

Figure 5 shows different views of a prototype of the probe of the present invention fabricated by the present inventors: Layout of a 4x8 rearranged into a $(4 \times 8) \times (8 / 8)$ intra-cortical shank with a width of the metalized area of $93\mu m$ (left and top inset). Optical micrograph of the fabricated neural probe. The central image shows a zoom into the tip, with the 3 metal levels (0, 1, and 2) marked with solid, dotted and dashed lines respectively.

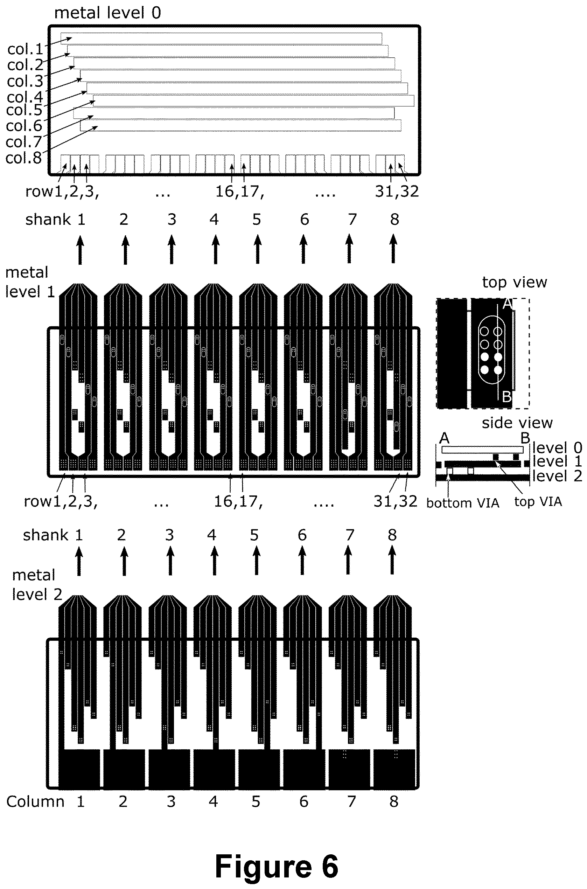

Figure 6: Schematic of the interconnection at the base of the probe of the present invention, for an embodiment for which the probe is an 8-shank intra-cortical neural probe with 4x8 active sensors per shank portion. The solid-line frame indicates the same area for the 3 metal levels. In the metal 0 level, the interconnections between the 8 column lines is defined. In the metal 1 level, the 32 tracks corresponding to the 32 rows of the array are defined. The connectivity through metal tracks defined in Figure 2a is used to connect the metal level 0 with the metal level 2 through the metal level 1. The inset on the right shows the interconnection between metal level 0 and metal level 2 that passes through the lines in metal level 1. The side view shows the profile of the 3 metal levels and the VIAs between point A and B. Finally, in the metal level 2 the metal tracks for the 8 columns of the array are defined. The first six columns enter in the shanks at the metal level 2 while the columns 7 and 8 are connected to the two central tracks of each shank in metal level 1.

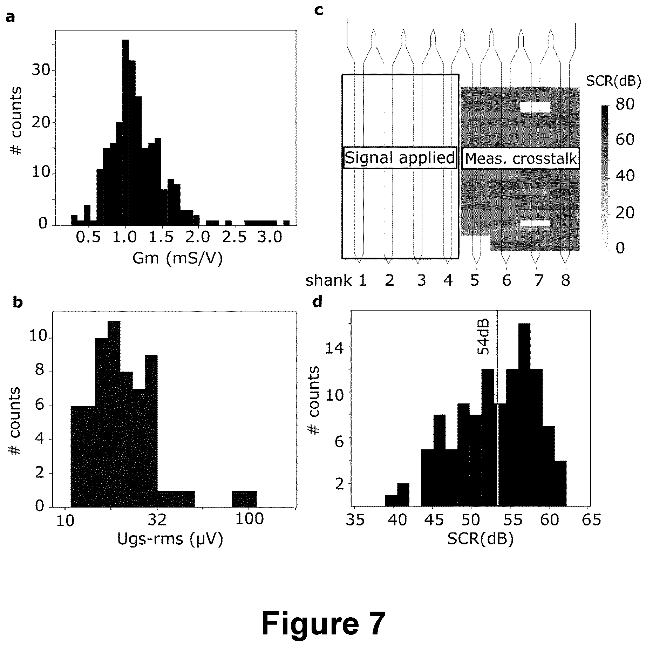

Figure 7: a. Histogram showing the transconductance ($G_m$) of 256 g-SGFETs in 8 shanks, where each of the shank presents the layout in Figure 5 and the interconnection between shanks is as schematized in Figure 6. b, The equivalent noise at the gate $U_{gs\text{-}rms}$ for 64 g-SGFETs in two independent shanks is shown. These two 1-shank devices were randomly selected from a wafer with 20 probes defined according to the layout in Figure 5. c, Measurement of crosstalk level among g-SGFETs in an 8-shank device. The solid line in the background illustrates the 8-shank probe. The signal was applied on 128 g-SGFETs (left square) and the crosstalk obtained from the other 128 g-SGFETs. The signal in the second group of 128 g-SGFETs was also measured by applying a signal selectively on their gates in order to calculate the signal to crosstalk ratio (grayscale map). The sites in white correspond to devices with g-SGFET with a Gm below a threshold of 0.5 mS/V. which were excluded to calculate the SCR. d, The SCR for g-SGFETs in panel c is shown in a histogram. The design target of 52dB is labelled with a vertical line. A certain dispersion around the design target is observed due to variability in the signal amplification or $G_m$.

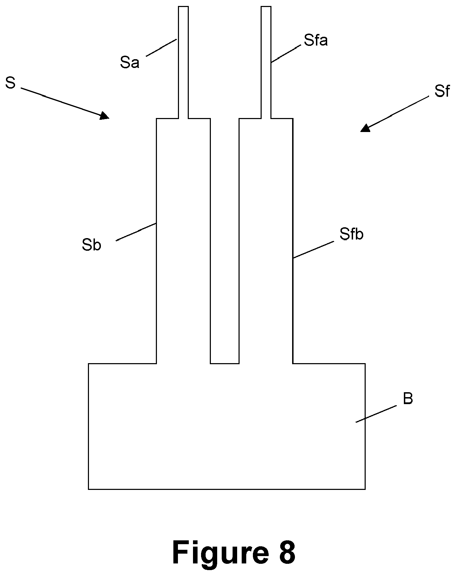

Figure 8 is a schematic view of the flexible support of the probe of the present invention for an embodiment for which the probe comprises two shank portions, each with a distal free end and a wider proximal free end, and a base portion attached to both proximal ends.

DESCRIPTION OF THE PREFERRED EMBODIMENTS

In the present section some working embodiments of the present invention will be described in detail.

The metal lines shown in the drawings implement the above mentioned electrically conductive tracks and electrically conductive connections. The elements identified as metal levels in the Figures refer to metal layers placed at the indicated depth level in the stack of the corresponding shaft portion, which are used to implement any electrically conductive path of the probe, including the above mentioned electrically conductive tracks, connections and interconnections.

The elements identified as VIA in the drawings refer to the above mentioned via holes filled with an electrically conductive material in order to make the indicated connection.

For the illustrated embodiments, in order to implement $m \times n$ sensor arrays in a linear (mxn)x(n/n) array the sensors are located in a layer (at level 0) above the metal lines that interconnect the sensors. The underlying tracks can then be connected to each particular sensor through VIA holes in the flexible insulating interlayers (see Fig. 1b). The underlying metal levels may contain more than 1 metal line (Fig. 1c and 1d). Following this approach, column and row metal lines do not cross and can be therefore integrated in the same metal layer (see Fig. 1d), in contrast to surface multiplexed probes with two-dimensional arrays. With this connectivity, an arbitrary number of columns and rows can be used to produce an array of (mxn)x(n/n) sensors (single row) or $(m \times j) \times (n/j)$, distributed in 1 or multiple (k) shanks. Moreover, this arrangement allows the stacking of multiple levels separated by insulating interlayers, providing more space for the metal lines. The distribution of the metal lines minimizes the shank width and/or the resistance of the conductive lines. For the illustrated embodiment, i.e. intra-cortical probes, the cross-section must be as small as possible to minimize tissue damage(9), with a width typically constrained to <100$\mu$m. On the other hand, for the illustrated embodiment, the track resistance must be below 50$\Omega$ to have a signal-to-crosstalk ratio (SCR) >36dBs (1, *10*), which has been shown low enough for arrays of at least 4x8 graphene field-effect transistors(*1*). Having highly conductive lines (e.g. made of Au, $\sigma_{Au}$ = 4,42 . $10^7 S/m$) with a thickness of $t \approx 300$nm and typical shank lengths in the range [3-10]mm, which allow to target deep brain structures of rodents and all cortical layers in humans, the width of the tracks must be in the range of [4.5-15] $\mu$m depending on the shank length. Having a separation of 3 $\mu$m between lines, which is a safe value for basic photolithography equipment used to fabricate the devices, the width of the shanks must be < [(4.5 $\mu$m +3 $\mu$m)N - (15 $\mu$m +3 $\mu$m)N] depending on the shank length, where N is the number of metal lines per level. For a typical shank length of 4mm, used to reach the hippocampus of a rat and most cortical layers in humans, a width of 100 $\mu$m and a maximum number of 6 metal lines can be integrated in a metal level while complying with a SCR>36dB. Having multiple metal levels, it is possible to increase the number of metal lines in the array. However, using VIAs through multiple interlayers constrains the space available for the metal tracks. VIAs present a minimal diameter in the range of the interlayer thickness (~2 $\mu$m for tested devices based on polyimide). In addition, a tolerance of 2 $\mu$m per side must be considered to prevent alignment errors as well as 3 $\mu$m spacing to neighbour metal lines, which amounts to an additional ~9 $\mu$m.

If the VIAs from metal level 2 (or higher) connect to metal layer 0 they take space from the metal lines in level 1 (or higher), as shown in the embodiment of Fig. 2a, where an intermediate electrically conductive connection Ci is placed surrounded by metal line of level 1, and connected to the metal lines of levels 2 and 0 through bottom and top VIAs.

Instead, the VIAs between metal layer 2 and layer 0 can be all aligned in the same position on the $\times$ axis of the shank to avoid any narrowing in metal lines in level 1, as shown in the embodiment of Fig. 2b, where the intermediate electrically conductive connection is spatially shifted and electrically insulated from the metal line also running at level 1, and connected to the metal lines of levels 2 and 0 through bottom and top VIAs.

This alignment of VIAs connecting metal level 0 and level 2 or deeper can be combined with the grouping of consecutive sensors connected to a same row. Using this grouping, some conductive lines can be ended closer to the base of the probe, therefore leaving additional space available closer to the tip of the shank, as shown for the embodiment of Fig. 3). Doing the grouping in the level 2 or deeper allows to connect all rows though VIAs that are in the same position on the x-axis (see Fig. 3). Assigning some of the metal lines in level 1 to the rows (connected to grouped sensors) there is space available in level 1 for VIAs connecting level 0 and level 2 or deeper.

Following this approach, an optimal design for multiplexed intra-cortical shanks can be derived comprising of 2 metal layers underneath layer 0, with a width of 93 $\mu$m and 12 metal lines (6 in level 1 and 6 in level 2). The connectivity of the rows can be grouped in neighbour active sensors as sketched in Fig. 1b, leaving more space for the metal lines in the tip of the shanks. In order to take additional advantage of metal lines that are ended close to the base of the probe, it is possible to make arrays with more rows than columns. For example, an array of 4x8 active sensors based on 2 levels of 6 metal tracks. Two of the metal tracks in the level 1 (acting as rows) can be connected to the corresponding sensors at the base of the shank, providing more space for the 4 remaining lines (acting as columns) to reduce their resistance.

Having established the design rules for optimal narrow intra-cranial, multiplexed, flexible shanks, parallel shanks can be integrated, with a second level of multiplexing. In this way, k shanks with mxn sensors each can be connected in parallel by adding only kxm lines, instead of k(mxn), as shown for the embodiment of Fig. 4. The wiring between common rows in multiple shanks can be done at the base of the flexible probe. In order to connect common rows among multiple shanks, metal lines that are not parallel to the shanks must be used. These metal lines must be laid out in a metal level different from the levels used for the columns and rows in each of the shanks. If the level 0, where the active sensors are defined, is not used to include row and column lines, this level can be used for the interconnection at the base (see Fig. 4). The resistance of metal tracks used for the interconnection between columns in multiple shanks can cause crosstalk between active sensors in different shanks. To minimize crosstalk, their resistance must be minimized. The impact of crosstalk is expected to increase with the number of sensors. In the case of metal lines in shanks (e.g. with 4x8 active sensors) a 36dB SCR was found to have a low impact for the detection of local field potentials (which present correlated signals in many sensors). The crosstalk induced by correlated components of signals in multiple sensors adds up linearly. Therefore, when adding shanks in parallel, the parasitic resistance of their interconnections must be lower than within single shanks to have a similarly low impact of crosstalk on signal quality. k shanks multiply the impact of crosstalk by a maximum of a k factor (i.e. $20*log10(k)$ dBs) in case of highly correlated signals. Thus, the resistance of interconnections must be a k factor smaller to ensure a low impact of crosstalk on signal integrity. For a multi-shank probe with 8 shanks, each with 32 active sensors (i.e. 256 sensors in total), the resistance of the interconnections must be $-50/8 \approx 6.25\Omega$, leading to a predicted SCR > 54dB. Therefore, connections with a thickness (t) of ~300nm thick Au, must present an aspect ratio (length-to-width L/W < 82).

In an interconnection such as the one sketched in Fig. 4, increasing the width of perpendicular lines ($w_{\perp}$) implies a longer length of parallel lines. The additional length of parallel lines is equal to the number of columns and rows interconnected among shanks ($i$) times ($w_{\perp}+s=W_{\perp}$), where s is the separation between metal lines. If the distance between shanks is d, the resistance of the interconnection is:

$$R = \frac{\rho}{t}\left(\frac{kd}{w_{\perp}} + \frac{W_{\perp}i}{w_{\parallel}}\right)$$

Eq. 1

Where p is the resistivity of the material used for the conductive lines. The first term on the right hand side of Eq. 1 represents the resistance of the perpendicular lines and the second term the resistance of the parallel lines. The latter can be expressed as a function of the space taken by perpendicular lines $W_{\perp}$ and the number of perpendicular lines $i$. Furthermore, the $w_{\parallel}$ is optimum when the parallel lines fill all the width of the said base portion (i.e. $W_{\parallel} = w_{\parallel} + s = d/N$), where $N$ is the number of metal lines per metal level in each shank. Rewriting Eq. 1 as:

$$R = \frac{\rho}{t}\left(\frac{kd}{w_{\perp}} + \frac{(s + w_{\perp})j}{(d/N) - s}\right)$$

Eq. 2

the dependence of R on interconnection geometrical factors is expressed explicitly. Eq. 2 can be used to find the optimal parameters that minimize the resistance. Assuming that s is much smaller than d/N and $w_{\perp}$, the interconnections resistance can be minimized by finding the optimal $w_{\perp}$ as:

$$\frac{dR}{dw_{\perp}} = 0 = \frac{-kd}{w_{\perp}^2} + \frac{jN}{d} \rightarrow w_{\perp} = d\sqrt{\frac{k}{jN}}$$

Making the width of lines parallel wider than this value does not contribute to decrease the interconnection resistance.

In order to demonstrate the concepts presented above, high sensor-count neural probes were fabricated and characterized. The produced devices are based on graphene solution-gated field-effect transistors on a polyimide probe using Au metal tracks of 300nm with an adhesion promoter layer of Ti (10nm) and a Ni interlayer (20nm) at the graphene-metal contact areas to reduce contact resistance. Single shanks of 4x8 g-SGFETs in the metal level 0, with 6 metal lines in level 1 and 6 metal lines in level 2. The 6 metal lines in level 2 comprise 6 rows. The 2 central metal lines in level 1 comprise 2 more row lines, while the 4 remaining metal lines on level 1 comprise the 4 columns. Each of the rows in level 2 is connected to consecutive graphene sensors, in order to enable the proper aligning of VIAs from the level 2 to level 0. Each of the 2 rows in level 1 are also connected to consecutive graphene sensors at the base of the shank, leaving an empty space in the level 1 of the remaining shank, where the VIAs from level 2 to level 0 can be defined. VIAs with a diameter of 8 $\mu$m have been used, filling the space left by the two central lines in level 1 (see Fig. 5). For 8-shank multiplexed intra-cortical probes, the 8 columns of each shank have been interconnected at the base of the probe as shown in Fig. 6. The devices fabricated confirm the suitability of g-SGFETs in terms of sensitivity and response in multiplexed operation (Fig. 7).

Fig. 7a presents an histogram of the $G_m$ in a 32x8 (256) g-SGFET array in an 8-shank probe, where each of the shanks contains 4x8 active sensors as defined in Fig. 5. Fig. 7b shows the characterization of two 1-shank probes with 4x8 g-SGFETs each, showing a good performance of the g-SGFETs in terms of equivalent noise at the gate ($U_{gs\text{-}rms}$), with a high sensitivity for a size of 25$\mu$m $\times$ 25$\mu$m compared to other multiplexed active sensors arrays on flexible substrates(12). Furthermore, the measured SCR among shanks meets the requirements of 54dB, with a certain dispersion around this value due to variability in the $G_m$ among transistors and variability in their resistance(13).

A person skilled in the art could introduce changes and modifications in the embodiments described without departing from the scope of the invention as it is defined in the attached claims.

References:

1. R. Garcia-Cortadella, N. Schafer, J. Cisneros-Fernandez, L. Re, X. Illa, G. Schwesig, A. Moya, S. Santiago, G. Guirado, R. Villa, A. Sirota, F. Serra-Graells, J. A. Garrido, A. Guimerà-Brunet, Switchless Multiplexing of Graphene Active Sensor Arrays for Brain Mapping. Nano Lett. 20, 3528-3537 (2020).

2. J. Viventi, D. H. Kim, L. Vigeland, E. S. Frechette, J. A. Blanco, Y. S. Kim, A. E. Avrin, V. R. Tiruvadi, S. W. Hwang, A. C. Vanleer, D. F. Wulsin, K. Davis, C. E. Gelber, L. Palmer, J. Van Der Spiegel, J. Wu, J. Xiao, Y. Huang, D. Contreras, J. A. Rogers, B. Litt, Flexible, foldable, actively multiplexed, high-density electrode array for mapping brain activity in vivo. Nat. Neurosci. 14, 1599-1605 (2011).

3. J. J. Jun, N. A. Steinmetz, J. H. Siegle, D. J. Denman, M. Bauza, B. Barbarits, A. K. Lee, C. A. Anastassiou, A. Andrei, Ç. Aydin, M. Barbic, T. J. Blanche, V. Bonin, J. Couto, B. Dutta, S. L. Gratiy, D. A. Gutnisky, M. Häusser, B. Karsh, P. Ledochowitsch, C. M. Lopez, C. Mitelut, S. Musa, M. Okun, M. Pachitariu, J. Putzeys, P. D. Rich, C. Rossant, W. L. Sun, K. Svoboda, M. Carandini, K. D. Harris, C. Koch, J. O'Keefe, T. D. Harris, Fully integrated silicon probes for high-density recording of neural activity. Nature. 551, 232-236 (2017).

4. A. Obaid, M. E. Hanna, Y. W. Wu, M. Kollo, R. Racz, M. R. Angle, J. Müller, N. Brackbill, W. Wray, F. Franke, E. J. Chichilnisky, A. Hierlemann, J. B. Ding, A. T. Schaefer, N. A. Melosh, Massively parallel microwire arrays integrated with CMOS chips for neural recording. Sci. Adv. 6, eaay2789 (2020).

5. M. Choi, Y. J. Park, B. K. Sharma, S.-R. Bae, S. Y. Kim, J.-H. Ahn, Flexible active-matrix organic light-emitting diode display enabled by MoS2 thin-film transistor. Sci. Adv. 4, eaas8721 (2018).

6. MicroFlex Array |, (available at https://www.blackrockmicro.com/microflex-array/).

7. E. Musk, Neurolink, An integrated brain-machine interface platform with thousands of channels. bioRxiv, 703801 (2019).

8. J. E. Chung, H. R. Joo, J. L. Fan, D. F. Liu, A. H. Barnett, S. Chen, C. Geaghan-Breiner, M. P. Karlsson, M. Karlsson, K. Y. Lee, H. Liang, J. F. Magland, J. A. Pebbles, A. C. Tooker, L. F. Greengard, V. M. Tolosa, L. M. Frank, High-Density, Long-Lasting, and Multi-region Electrophysiological Recordings Using Polymer Electrode Arrays. Neuron. 101, 21-31.e5 (2019).

9. F. He, R. Lycke, M. Ganji, C. Xie, L. Luan, Ultraflexible Neural Electrodes for Long-Lasting Intracortical Recording. iScience. 23, 101387 (2020).

10. N. Schaefer, R. Garcia-Cortadella, J. Martínez-Aguilar, G. Schwesig, X. Illa, A. M. Lara, S. Santiago, C. Hébert, G. Guirado, R. Villa, A. Sirota, A. Guimerà-Brunet, J. A. Garrido, Multiplexed neural sensor array of graphene solution-gated field-effect transistors. 2D Mater. 7, 025046 (2020).

11. R. Garcia-Cortadella, G. Schwesig, C. Jeschke, X. Illa, A. L. Gray, S. Savage, E. Stamatidou, I. Schiessl, E. Masvidal-Codina, K. Kostarelos, A. Guimerà-Brunet, A. Sirota, J. A. Garrido, Graphene active sensor arrays for long-term and wireless mapping of wide frequency band epicortical brain activity. Nat. Commun. 12, 1-17 (2021).

12. C. H. Chiang, S. M. Won, A. L. Orsborn, K. J. Yu, M. Trumpis, B. Bent, C. Wang, Y. Xue, S. Min, V. Woods, C. Yu, B. H. Kim, S. B. Kim, R. Huq, J. Li, K. J. Seo, F. Vitale, A. Richardson, H. Fang, Y. Huang, K. Shepard, B. Pesaran, J. A. Rogers, J. Viventi, Development of a neural interface for high-definition, long-term recording in rodents and nonhuman primates. Sci. Transl. Med. 12 (2020), doi:10.1126/scitranslmed.aay4682.

13. R. Garcia-Cortadella, N. Schafer, J. Cisneros-Fernandez, L. Re, X. Illa, G. Schwesig, A. Moya, S. Santiago, G. Guirado, R. Villa, A. Sirota, F. Serra-Graells, J. A. Garrido, A. Guimerà-Brunet, Nano Lett., in press, doi:10.1021/acs.nanolett.0c00467.

Claims

1. A flexible probe, comprising:

   - an electrically insulating flexible support comprising at least one shank portion (S);
   - a plurality of active elements arranged on said at least one shank portion (S); and
   - a plurality of tracks (T) electrically connected to terminals of said plurality of elements;

   **characterized in that:**

   - said at least one shank portion (S) comprises at least a first electrically insulating layer with a top face and a bottom face, wherein the active elements of said plurality of active elements are arranged on said top face of said first electrically insulating layer (PI); and
   - at least one electrically conductive track of said plurality of electrically conductive tracks (T) is arranged on said bottom face of the first electrically insulating layer (PI), running at a first depth level, and is electrically connected to a terminal of at least one of said plurality of active elements through a via hole defined in at least the first electrically insulating layer (PI); and
   - at least part of the plurality of electrically conductive tracks (T) are electrically interconnected to implement a multiplexing technique.

2. The flexible probe according to claim 1, wherein said at least part of the plurality of electrically conductive tracks (T) are electrically interconnected so that with m + n electrically conductive tracks (T) the active elements are addressed in an $(m \times j) \times (n / j)$ array, where n is a multiple of j, where m, n and j are positive integers.

3. The flexible probe according to claim 1, wherein said electrically insulating flexible support comprises at least one further shank portion (Sf), and wherein the flexible probe further comprises:

   - a further plurality of active elements arranged on said further shank portion (Sf); and
   - a further plurality of electrically conductive tracks (Tf) electrically connected to terminals of said further plurality of active elements;

   wherein:

   - said at least one further shank portion (Sf) comprises at least a first further electrically insulating layer (Plf) with a top face and a bottom face, wherein the active elements of said further plurality of active elements are arranged on said top face of said first further electrically insulating layer;
   - at least one electrically conductive track of said further plurality of electrically conductive tracks (Tf) is arranged on said bottom face of the first further electrically insulating layer (Plf), running at a first depth level, and is electrically connected to a terminal of at least one of said further plurality of active elements through a via hole defined in at least the first further electrically insulating layer (Plf); and
   - at least part of the further plurality of electrically conductive tracks (Tf) are electrically interconnected to implement a multiplexing technique.

4. The flexible probe according to claim 1, 2 or 3, wherein:

   - the at least one shank portion (S) comprises a stack of electrically insulating layers (PI), including at least said first electrically insulating layer (PI) on top of at least a second electrically insulating layer (PI);

   and/or

   - the at least one further shank portion (Sf) comprises a stack of electrically insulating layers (PI), including at least said first further electrically insulating layer (Plf) on top of at least a second further electrically insulating layer (Plf).

5. The flexible probe according to claim 3 or 4, wherein at least part of the plurality of electrically conductive tracks (T) are interconnected with at least part of the further plurality of electrically conductive tracks (Tf), to implement a multiplexing technique, wherein the electrically insulating flexible support comprises a base portion (B) from which the at least one shank portion (S) and the at least one further shank portion (Sf) project, wherein said interconnection of at least part of the plurality of electrically conductive tracks (T) with at least part of the further plurality of electrically conductive tracks (Tf) is made on said base portion (B).

6. The flexible probe according to claim 4, wherein the at least one shank portion (S) and/or the at least one further shank portion (Sf) has a distal free end part (Sa, Sfa) and a proximal end part (Sb, Sfb) that is wider than said distal free end part (Sa, Sfa), wherein said proximal end part (Sb, Sfb) is joined to the base portion (B).

7. The flexible probe according to any of the previous claims, wherein at least two electrically conductive tracks of said plurality (T) and/or further plurality (Tf) of electrically conductive tracks run, at the same depth level, said first depth level, and are electrically connected to at least two respective terminals of at least one active element of said plurality and/or of said further plurality of active elements through at least two respective via holes defined in at least the first electrically insulating layer (PI) and/or the first further electrically insulating layer (PIf).

8. The flexible probe according to claim 4 or to any of claims 5 to 7 when depending on claim 4, wherein:

- said at least one electrically conductive track of said plurality of electrically conductive tracks (T) runs at said first depth level, in the stack of the shank portion (S), and wherein at least another electrically conductive track of the plurality of electrically conductive tracks (T) is arranged on a bottom face of the second electrically insulating layer (PI), running at a second depth level in the stack of the shank portion (S), and is electrically connected to a terminal of at least one active element of the plurality of active elements through at least two via holes respectively defined in at least the first and second electrically insulating layers (PI); and/or
- said at least one electrically conductive track of said further plurality of electrically conductive tracks (Tf) runs at said first depth level, in the stack of the further shank portion (Sf), and wherein at least another electrically conductive track of the further plurality of electrically conductive tracks (Tf) is arranged on a bottom face of the second further electrically insulating layer (PIf), running at a second depth level in the stack of the further shank portion (Sf), and is electrically connected to a terminal of at least one active element of the further plurality of active elements through at least two via holes respectively defined in at least the first and second further electrically insulating layers (PIf).

9. The flexible probe according to claim 8, further comprising at least one intermediate electrically conductive connection (Ci) arranged, at said first depth level, between the first and second electrically insulating layers (PI) and/or between the first and second further electrically insulating layers (PIf), and which is electrically connected, through said at least two via holes, to said terminal of at least one active element (A, Af) and to the electrically conductive track (T, Tf) running at the second depth level of the stack of the shank portion (S) and/or of the further shank portion (Sf).

10. The flexible probe according to claim 9, wherein said at least one intermediate electrically conductive connection (Ci) is spatially shifted and electrically insulated from said at least one electrically conductive track (T, Tf) also running at the first depth level.

11. The flexible probe according to any of the previous claims, constituting an intracranial neural probe.

12. The flexible probe according to claim 11, wherein:

- said intracranial neural probe is an epicortical neural probe, wherein:

  - the shank portion (S) and/or further shank portion (Sf), or said distal free end part (Sa, Sfa) thereof, has a width below 1.5 mm and a length between 20 and 100 mm; and
  - each electrically conductive track (T, Tf) has an electrical resistance below 50 $\Omega$, a maximum thickness of 500 nm, a width ranging between 4.5 $\mu$m and 15 $\mu$m, and a separation of at least 3 $\mu$m with respect to another electrically conductive track;
  or

- said intracranial neural probe is a depth neural probe, wherein:

  - the shank portion (S) and/or further shank portion (Sf), or said distal free end part (Sa, Sfa) thereof, has a width below 150 $\mu$m and a length ranging between 3 mm and 10 mm; and
  - each electrically conductive track (T, Tf) has an electrical resistance below 50 $\Omega$, a maximum thickness of 500 nm, a width ranging between 4.5 $\mu$m and 15 $\mu$m, and a separation of at least 3 $\mu$m with respect to

another electrically conductive track (T, Tf).

13. The flexible probe according to any of the previous claims, constituting a depth neural probe, and wherein the flexible support is dimensioned, shaped, and flexible enough so that it can be rolled around a three-dimensional structure.

14. The flexible probe according to claim 5 or to any of claim 6 or 13 when depending on claim 5, wherein said base portion (B) also comprises a stack of at least first and second electrically insulating layers, and wherein the interconnection of at least part of the plurality of electrically conductive tracks (T) with at least part of the further plurality of electrically conductive tracks (Tf) is made by means of interconnection electrically conductive tracks (Ti) arranged at a depth level, in said base portion stack, that is different to the depth level at which the electrically conductive tracks (T, Tf) run, in the shank portion (S) and further shank portion (Sf), respectively.

15. The flexible probe according to any of the previous claims, wherein said active elements are transistors, wherein the drain and source terminals of said transistors are electrically connected to said electrically conductive tracks (T, Tf), so that the transistors are arranged in an addressable matrix with m columns and $n$ rows, where the electrically conductive tracks for the m columns address the common source or drain terminal of all transistors in each column, and the $n$ rows address either the common drain or source terminal or the common gate terminal of all transistors in each row, to implement said multiplexing technique.

**a**

Planar array
**(top-view)**

PI-substrate

1   2

3   4

col. 1   col. 2   row 1   row 2

y

x

**b**

Intra-cortical
**(side-view)**

4

3

2

y  1

z

Metal1  Metal2  Metal3  Metal4

PI-interlayers

**c**

metal level 0   metal level 1

4

3

2

y  1

z

PI-interlayer

**d**

metal level 0   active channel

B

VIA

C

A

PI

metal level 1
T

**e**

metal level 0   active channel

C

VIA   A

B

metal level 1

side view

# Figure 1

**a**

**b**

# Figure 2

# Figure 3

**Figure 4**

**Figure 5**

metal level 0

col.1
col.2
col.3
col.4
col.5
col.6
col.7
col.8

row1,2,3,          ...          16,17,          ....          31,32

shank 1     2     3     4     5     6     7     8

metal
level 1

top view

A

B

row1,2,3,          ...          16,17,          ....          31,32

side view

A          B
level 0
level 1
level 2

bottom VIA     top VIA

shank 1     2     3     4     5     6     7     8

metal
level 2

Column  1     2     3     4     5     6     7     8

# Figure 6

Figure 7

**Figure 8**

| Europäisches Patentamt European Patent Office Office européen des brevets | **EUROPEAN SEARCH REPORT** | Application Number EP 22 38 2017 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/193663 A1 (DELIGIANNI HARIKLIA [US] ET AL) 12 July 2018 (2018-07-12) * paragraphs [0005], [0006], [0071], [0073], [0074], [0105], [0130]; figures 1,19 * | 1,4-12, 15 | INV. A61N1/05 A61B5/293 A61B5/00 |
| X | EP 3 111 835 A1 (IMEC VZW [BE]; UNIV LEUVEN KATH [BE]) 4 January 2017 (2017-01-04) * paragraphs [0025], [0042], [0070], [0071], [0077], [0097], [0098], [0104]; figures 1, 5 * | 1-3,5,6, 11-13,15 | |
| X | US 2020/170533 A1 (YU ROY R [US] ET AL) 4 June 2020 (2020-06-04) * paragraphs [0004] - [0007], [0038], [0051] - [0055]; figures 1,2,7 * | 1-3,11, 12,14,15 | |
| X | EP 2 353 636 A1 (MAX PLANCK GESELLSCHAFT [DE]) 10 August 2011 (2011-08-10) * the whole document * | 1 | |
| X | US 2013/030274 A1 (JAMIESON BRIAN [US] ET AL) 31 January 2013 (2013-01-31) * the whole document * | 1 | TECHNICAL FIELDS SEARCHED (IPC) A61N A61B |
| X | US 2018/078162 A1 (HAN SHU-JEN [US]) 22 March 2018 (2018-03-22) * the whole document * | 1 | |

The present search report has been drawn up for all claims

| Place of search | Date of completion of the search | Examiner |
|---|---|---|
| Munich | 30 May 2022 | Pfeiffer, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 38 2017

This annex lists the patent family members relating to the patent documents cited in  the above-mentioned European search report.
The members are as contained in the European Patent Office EDP file on
The European Patent Office is in no way liable for these particulars which are merely given for the purpose of information.

30-05-2022

| Patent document cited in search report | | Publication date | Patent family member(s) | | Publication date |
|---|---|---|---|---|---|
| US 2018193663 | A1 | 12-07-2018 | NONE | | |
| EP 3111835 | A1 | 04-01-2017 | EP | 3111835 A1 | 04-01-2017 |
| | | | US | 2017000368 A1 | 05-01-2017 |
| US 2020170533 | A1 | 04-06-2020 | NONE | | |
| EP 2353636 | A1 | 10-08-2011 | NONE | | |
| US 2013030274 | A1 | 31-01-2013 | CA | 2842950 A1 | 31-01-2013 |
| | | | EP | 2736407 A2 | 04-06-2014 |
| | | | US | 2013030274 A1 | 31-01-2013 |
| | | | US | 2014200431 A1 | 17-07-2014 |
| | | | US | 2018028119 A1 | 01-02-2018 |
| | | | WO | 2013016350 A2 | 31-01-2013 |
| US 2018078162 | A1 | 22-03-2018 | US | 2018078162 A1 | 22-03-2018 |
| | | | US | 2018078193 A1 | 22-03-2018 |
| | | | US | 2018078194 A1 | 22-03-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **R. GARCIA-CORTADELLA ; N. SCHAFER ; J. CISNEROS-FERNANDEZ ; L. RE ; X. IIIA ; G. SCHWESIG ; A. MOYA ; S. SANTIAGO ; G. GUIRADO ; R. VILLA.** Switchless Multiplexing of Graphene Active Sensor Arrays for Brain Mapping. *Nano Lett,* 2020, vol. 20, 3528-3537
- **J. VIVENTI ; D. H. KIM ; L. VIGELAND ; E. S. FRECHETTE ; J. A. BLANCO ; Y. S. KIM ; A. E. AVRIN ; V. R. TIRUVADI ; S. W. HWANG ; A. C. VANLEER.** Flexible, foldable, actively multiplexed, high-density electrode array for mapping brain activity in vivo. *Nat. Neurosci.,* 2011, vol. 14, 1599-1605
- **J. J. JUN ; N. A. STEINMETZ ; J. H. SIEGLE ; D. J. DENMAN ; M. BAUZA ; B. BARBARITS ; A. K. LEE ; C. A. ANASTASSIOU ; A. ANDREI ; Ç. AYDIN.** Fully integrated silicon probes for high-density recording of neural activity. *Nature,* 2017, vol. 551, 232-236
- **A. OBAID ; M. E. HANNA ; Y. W. WU ; M. KOLLO ; R. RACZ ; M. R. ANGLE ; J. MÜLLER ; N. BRACKBILL ; W. WRAY ; F. FRANKE.** Massively parallel microwire arrays integrated with CMOS chips for neural recording. *Sci. Adv.,* 2020, vol. 6
- **M. CHOI ; Y. J. PARK ; B. K. SHARMA ; S.-R. BAE ; S. Y. KIM ; J.-H. AHN.** Flexible active-matrix organic light-emitting diode display enabled by MoS2 thin-film transistor. *Sci. Adv.,* 2018, vol. 4, eaas8721
- **E. MUSK.** Neurolink, An integrated brain-machine interface platform with thousands of channels. *bioRxiv,* 2019, 703801

- **J. E. CHUNG ; H. R. JOO ; J. L. FAN ; D. F. LIU ; A. H. BARNETT ; S. CHEN ; C. GEAGHAN-BREINER ; M. P. KARLSSON ; M. KARLSSON ; K. Y. LEE.** High-Density, Long-Lasting, and Multi-region Electrophysiological Recordings Using Polymer Electrode Arrays. *Neuron,* 2019, vol. 101, 21-31
- **F. HE ; R. LYCKE ; M. GANJI ; C. XIE ; L. LUAN.** Ultraflexible Neural Electrodes for Long-Lasting Intracortical Recording. *iScience,* 2020, vol. 23, 101387
- **N. SCHAEFER ; R. GARCIA-CORTADELLA ; J. MARTÍNEZ-AGUILAR ; G. SCHWESIG ; X. IIIA ; A. M. LARA ; S. SANTIAGO ; C. HÉBERT ; G. GUIRADO ; R. VILLA.** Multiplexed neural sensor array of graphene solution-gated field-effect transistors. *2D Mater,* 2020, vol. 7, 025046
- **R. GARCIA-CORTADELLA ; G. SCHWESIG ; C. JESCHKE ; X. IIIA ; A. L. GRAY ; S. SAVAGE ; E. STAMATIDOU ; I. SCHIESSL ; E. MASVIDAL-CODINA ; K. KOSTARELOS.** Graphene active sensor arrays for long-term and wireless mapping of wide frequency band epicortical brain activity. *Nat. Commun.,* 2021, vol. 12, 1-17
- **C. H. CHIANG ; S. M. WON ; A. L. ORSBORN ; K. J. YU ; M. TRUMPIS ; B. BENT ; C. WANG ; Y. XUE ; S. MIN ; V. WOODS.** Development of a neural interface for high-definition, long-term recording in rodents and nonhuman primates. *Sci. Transl. Med.,* 2020, vol. 12
- **R. GARCIA-CORTADELLA ; N. SCHAFER ; J. CISNEROS-FERNANDEZ ; L. RE ; X. IIIA ; G. SCHWESIG ; A. MOYA ; S. SANTIAGO ; G. GUIRADO ; R. VILLA.** *Nano Lett.*